(19) Europäisches Patentamt
European Patent Office
Office européen des brevets

(11) Publication number: 0 066 922 B1

(12) **EUROPEAN PATENT SPECIFICATION**

(45) Date of publication of patent specification: **23.10.85**

(51) Int. Cl.⁴: **C 07 C 127/19,** C 07 C 127/15

(21) Application number: **82200632.6**

(22) Date of filing: **24.05.82**

(54) **Process for preparing asymmetric ureas from urethans and amines.**

(30) Priority: **02.06.81 IT 2208281**

(43) Date of publication of application:
**15.12.82 Bulletin 82/50**

(45) Publication of the grant of the patent:
**23.10.85 Bulletin 85/43**

(84) Designated Contracting States:
**AT BE CH DE FR GB LI LU NL SE**

(56) References cited:
**GB-A- 899 718**
**US-A-3 161 676**

(73) Proprietor: **ANIC S.p.A.**
**Via Ruggero Settimo, 55**
**I-90139 Palermo (IT)**

(72) Inventor: **Romano, Ugo**
**Via XXV Aprile 10**
**I-20059 - Vimercate (Milan) (IT)**
Inventor: **Fornasari, Giancarlo**
**Via Marco D'Agrate 19/A**
**I-20139 Milan (IT)**

(74) Representative: **Roggero, Sergio et al**
**Ing. Barzanò & Zanardo Milano S.p.A. Via Borgonuovo 10**
**I-20121 Milano (IT)**

**EP 0 066 922 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing certain asymmetric ureas, useful as active principles for pesticide and herbicide formulations, by reacting, in the presence of a catalyst, an amine and a carbamate, and is particularly directed to solving the problem of finding an efficient catalyst for such a reaction.

According to the closest prior art, US 3,161,676, the reaction in question was catalyzed by a Lewis' acid of metallic nature, the metals involved being tin, copper and aluminium. The process according to the invention is a process for preparing asymmetric ureas having the general formula RNH—CO—NR''R''', wherein R, R'' and R''' are alkyls or aryls, by catalytically reacting an amine having the general formula R''—NH—R''' with a carbamate having the formula RNH—CO—OR', wherein R' is an alkyl or an aryl, characterized in that an alkali metal alkoxide is used as the catalyst. The prior art mentioned above could not enable anyone skilled in the art to employ a basic catalyst such as an alkali metal alkoxide. It has been ascertained, instead, that not only the reaction takes place smoothly and takes less time, but the yields of the end product are very high as compared with those obtainable with the prior art procedures, the selectivity being also improved. Inasmuch as the general reaction pattern is known, the present specification will suggest, for the sake of completeness, several substituents and reaction parameters. The typical reaction pattern for preparing asymmetric ureas is:

$$RNH\underset{\underset{O}{\|}}{C}—OR'+R''R'''NH \rightarrow RNH\underset{\underset{O}{\|}}{C}NR''R'''+R'OH$$

and the aminolysis reaction proceeds with the formation of urea and alcohol.

By way of indication, the substituents R, R', R'', R''' can be of the following types:

R can be an alkyl or an aryl and, more particularly, the aryl can carry substituents at the nucleus. Therefore, R can be methyl, ethyl, n-propyl, isopropyl, n-butyl, isobutyl, cyclohexyl, cyclooctyl, phenyl, 3-chlorophenyl, 4-chlorophenyl, 4-methyl-3-chlorophenyl, 3,4-dichlorophenyl, 4-methoxy-3-chlorophenyl, 3-trifluoromethylphenyl, or 4-parachlorophenoxyphenyl.

R', the substituent at the carbamate oxygen, is generally an alkyl or a benzyl, such as methyl, ethyl, n-propyl, isopropyl, n-butyl, isobutyl, sec-butyl, benzyl or cyclohexyl. R'' and R''' are equal to, or different from, one another and can be alkyls and aryls (these latter can have substituents at the nucleus), such as methyl, ethyl, n-propyl, isopropyl, n-butyl, isobutyl, cyclohexyl, methoxy, phenyl, methylphenyl or chlorophenyl.

The amines used in the asymmetric urea synthesis reactions can be primary or secondary amines, aliphatic and/or aromatic amines, according to the type of substituent which is used. The catalyst to amine molar ratio varies from 1:5 to 1:100, and it is preferred, but not compulsory, that the alkoxy radical of the alkoxide catalyst is the same as that of the starting carbamate.

A practical rule for improving the yield of the expected product considerably, so approaching nearly the theoretical values, is to withdraw the alcohol as it is being produced in the reaction.

The invention will be better illustrated by the following examples, of which examples 1 and 2 are comparative examples in which no catalyst at all is used, whereas examples 3 to 10 inclusive show the best way to use the present invention.

Example 1

50 g of toluene, 13 g of di-n-butylamine (0.1 moles) and 8.5 g of ethyl carbanilate (0.0515 moles) are fed into a 150 cc stainless steel autoclave.

The autoclave is placed in a bath, temperature controlled at 165°C for six hours and is stirred continuously. At the end of the six hours, the autoclave is removed from the temperature controlled bath and allowed to cool. The autoclave is opened, and 15 cc of 18% HCl in water are added to the reaction mixture in order to separate the unreacted di-n-butylamine in the form of hydrochloride. The organic layer is then separated from the water, and the product is separated by fractional crystallisation from n-hexane. In this manner, a very voluminous flaky white solid is obtained (MP 80°C), its infrared (IR) spectrum and mass spectrum confirming that the product is 1-1-di-n-butyl-3-phenyl urea.

6.93 g of urea are obtained from the fractional crystallisation, equivalent to a yield of 54.3% and a carbanilate conversion of 65.5 mol%.

Example 2

14.2 g (0.2 moles) of diethylamine, 8.5 g (0.05 moles) of ethylcarbanilate, and 50 g of toluene are fed into the same autoclave, and the reaction system is heated to 165° for three hours. The procedure of example 1 is then followed in order to separate the reaction product.

A carbanilate conversion of 81.5 mol% is obtained, with an asymmetric urea yield of 62.8%.

Example 3

13 g of di-n-butylamine, 8.5 g of ethyl carbanilate, 0.18 g of sodium ethylate and 50 g of toluene are fed

into the autoclave. The mixture is kept at a temperature of 120°C for three hours. On termination of the reaction, the procedure of the preceding examples is followed in order to separate the final product.

The asymmetric urea yield is 74.6%, the remainder being unconverted product.

Example 4

The reaction was carried out in a 250 cc flask fitted with a thermometer, magnetic stirrer and a Vigreux fractionation column connected to a collection flask by way of a condenser. 13 g of di-n-butylamine, 8.5 g of ethyl carbanilate, 0.18 g of sodium ethylate and 50 g of toluene were fed in, and the mixture was reacted for 1.5 hours at 120°C. An asymmetric urea yield of 58.8% was obtained, with a carbanilate conversion of 60.6%.

Example 5

13 g of di-n-butylamine, 8.5 g of ethyl carbanilate, 0.18 g of sodium ethylate and 50 g of toluene are fed into the device illustrated in example 4. The mixture is kept at a temperature of 120°C for three hours in a temperature controlled bath.

However, the ethanol which forms is distilled off during the reaction, and the toluene and residual amine are removed under vacuum at the end in order to recover the product. This is crystallised in the usual manner from n-hexane, to obtain a product with a yield of 95%, the remainder being unconverted substance.

Example 6

7.8 g of di-n-butylamine, 8.58 g of ethyl carbanilate, 0,09 g of sodium ethylate and 50 g of toluene are fed into the system.

The experiment is carried out under the same conditions as example 5.

The final urea yield is 88%.

Example 7

6.51 g of cyclohexylamine, 8.54 g of ethyl carbanilate, 0.18 g of sodium ethylate and 60 g of toluene are fed into the system. The reaction mixture is kept in a bath, temperature controlled at 120°C for 2 hours 15 minutes.

As in the case of examples 5 and 6, the ethanol is distilled off as it forms in the reaction, and the toluene and unreacted amine are removed at the end under vacuum.

The final product obtained by fractional crystallisation as in the preceding examples was analysed by NMR, mass spectrometry and IR methods, from which the product was found to be N-phenyl-N'-cyclohexyl urea (MP 178—179°C).

The yield is 92% (as recrystallised product).

Example 8

The experiment of the preceding example is repeated, changing only the operating temperature which in this case was 100°C. A recrystallised product yield of 84% is obtained.

Example 9

Operating as in example 5, 11.5 g (0.0540 moles) of 3-chloro-4-methyl-ethylcarbanilate, 10.4 g (0.1031 moles) of di-n-propylamine, 0.18 g of sodium ethylate and 50.5 g of toluene are fed into the system.

The mixture is reacted at 115°C for 3 hours.

The carbanilate conversion was approximately 100%, with an asymmetric urea selectivity of 98%.

Example 10

Operating as in example 4, 4.92 g (0.028 moles) of N-cyclohexyl-ethylcarbamate, 7.43 g (0.057 moles) of di-n-butylamine, 0.68 g of 20% EtONa in EtOH and 30 g of toluene are fed into the system.

The mixture is reacted for 3 hours at 115°C and the ethanol is distilled off as it forms.

The carbamate conversion is 96%.

The asymmetric urea yield, as product recrystallised from n-hexane, is 88% (6.26 g) with M.P. of 70—71°C.

**Claims**

1. A process for preparing asymmetric ureas having the general formula RNH—CO—NR''R''', wherein R, R'' and R''' are alkyls or aryls, by catalytically reacting an amine having the general formula R''—NH—R''', with a carbamate having the general formula RNH—CO—OR', wherein R' is an alky or an aryl, characterized in that an alkali metal alkoxide is used as the catalyst.

2. Process according to claim 1, characterized in that the reaction temperature does not exceed 120°C.

3. A process according to claim 1, characterized in that said catalyst is sodium ethylate.

**Patentansprüche**

1. Verfahren zur Herstellung asymmetrischer Harnstoffe der allgemeinen Formel RNH—CO—NR''R''', worin R, R'' und R''' für Alkyl- oder Arylreste stehen, durch katalytische Umsetzung eines Amins der allgemeinen Formel R''—NH—R''' mit einem Carbamat der allgemeinen Formel RNH—CO—OR', worin R' einen Alkyl- oder einen Arylrest bedeutet, dadurch gekennzeichnet, daß eine Alkalimetallalkoxid als Katalysator verwendet wird.

2. Verfahren nach Anspruch 1, dadurchgekennzeichnet, daß die Umsetzungstemperatur 120°C nicht überschreitet.

3. Verfahren nach Anspruch 1, dadurch gekennzeichnet, daß der genannte Katalysator Natriummethylat ist.

**Revendications**

1. Procédé de préparation d'urées asymétriques ayant la formule générale RNH—CO—NR''R''', dans laquelle R, R'' et R''' sont des groupes alkyle ou aryle, en faisant réagir catalytiquement une amine ayant la formule générale R''—NH—R''' avec un carbamate ayant la formule générale RNH—CO—OR', dans laquelle R' est un groupe alkyle ou aryle, caractérisé par le fait qu'un alcoolate de métal alcalin est utilisé comme catalyseur.

2. Procédé selon la revendication 1, caractérisé par le fait que la température de réaction ne dépasse pas 120°C.

3. Procédé selon la revendication 1, caractérisé par le fait que ledit catalyseur est l'éthylate de sodium.